# EUROPEAN PATENT APPLICATION

(11) **EP 3 588 094 A1**
(43) Date of publication of application: **01.01.2020**
(21) Application number: 19182433.3
(22) Date of filing: 25.06.2019
(51) Int. Cl.: G01N 35/00, B03C 1/01, B03C 1/033, B03C 1/28, C12N 15/10, G01N 35/04

(54) **ROBOTIZED DEVICE FOR THE LOADING AND THE HANDLING OF A PLURALITY OF COATING ELEMENTS USABLE IN A MOLECULAR EXTRACTION PROCESS, IN PARTICULAR NUCLEIC ACIDS**

(30) Priority: 25.06.2018 IT 201800006621
(71) Applicant: MASMEC S.p.A., 70026 Modugno (IT)
(72) Inventor: LARIZZA, Pietro, 70026 MODUGNO (BA) (IT)
(74) Representative: Bongiovanni, Simone

(57) **Abstract**

A robotized device for the loading and the handling of a plurality of coating elements usable in a molecular extraction process, in particular DNA, comprising: a mobile head (5) arranged in a three-dimensional space with respect to a plane on which the device rests, under the force of actuators controlled by an electronic unit (7), a picking and conveying structure (9) carried by the head (5) adapted to pick/use/unload a plurality of single-use coating elements (10) each of which comprises a tubular element (10-t) adapted to coat an elongated element (12) belonging to an array of elements elongated from the picking and conveying structure.

## Description

### CROSS-REFERENCE TO RELATED APPLICATIONS

This patent application claims priority from Italian patent application no. 102018000006621 filed on June 25, 2018.

### TECHNICAL FIELD

The present invention concerns a robotized device for the loading and the handling of a plurality of coating elements usable in a molecular extraction process, in particular nucleic acids.

### PRIOR ART

As is known, the extraction of nucleic acids is the first step in molecular biology applications.

To extract a nucleic acid from a sample (entire organ, tissue, cell culture, blood, biological liquid, etc.), firstly the cell membrane of the cell samples is broken (lysis). The lysis operation can be performed using different techniques, for example by mechanical destruction of the cell membrane (hypotonic lysis), destruction of the cell membrane by means of enzymes or by destroying the cell membrane by means of chemical treatments (lysis with detergents).

Detergents cause rupture of the cell membrane and dissociate the proteins from the nucleic acids.

Following rupture of the cell walls and the membrane, a complex mixture is obtained consisting, for example, of lipid, mono and polysaccharide nucleic acids (DNA, RNA), proteins and salts.

Removal of the proteins from the mixture is particularly important since these can degrade the nucleic acids or bind with the nucleic acids preventing the subsequent steps of the process which entail purification and separation of the nucleic acids from the mixture.

According to a recent technology, the nucleic acid extraction process can be carried out by using micro-beads (microparticles) adapted to directly absorb DNA fragments from the solution containing the cells subject to lysis.

The micro-beads are released into the solution so that the nucleic acid molecules deposit on their surface and are subsequently collected using a permanent magnet since the micro-beads are made of a metal material which is magnetically attracted.

In further detail, the permanent magnet is coated by a tubular coating element of single-use type which is immersed in the solution containing the micro-beads so that the latter are attracted and deposit on the surface of the coating element.

Subsequently the coating element and the permanent magnet housed in it are extracted from the container containing the mixture so as to collect the micro-beads. The micro-beads normally undergo a washing step to eliminate contaminants and salts in which the coating element and the permanent magnet are arranged inside a container provided with a washing solution.

At the end of said washing operations, the permanent magnet is extracted from the coating element allowing release of the micro-beads on which fragments of DNA are present.

The European patent EP-B-2.848.943 by the same applicant describes a robotized device for the loading and the handling of a plurality of tubular covering elements usable in a molecular extraction process, in particular DNA, comprising a mobile head moving in a three-dimensional space with respect to a plane on which the device rests under the force of actuators controlled by an electronic unit and a picking and conveying structure carried by the head adapted to pick/use/unload the single-use coating elements each of which comprises a tubular element closed at a first end by a bottom wall and defining at a second end thereof a circular opening surrounded by a cylindrical annular edge.

The picking and conveying structure comprises a first upper structure directly carried by the head and a second lower structure mobile with respect to the first upper structure along a vertical guide interposed between the first and the second structures between a first stroke end position in which the first structure is arranged side-by-side with the second structure and a second stroke end position in which the lower structure is spaced with respect to the upper structure by a predetermined distance D.

A plurality of cylindrical elongated elements is carried by the upper structure and are arranged with first axes parallel and coplanar to one another so as to form an array of elongated elements spaced from one another by a centre-to-centre distance KK; each elongated element carries at one free end thereof a respective magnetic element.

A plurality of tubular connection sleeves is carried by the lower structure, and each is adapted to couple with a respective tubular element. A quick coupling/uncoupling device is interposed between the first upper structure and the second lower structure.

The robot is adapted to carry out a first positioning step of the head with respect to the coating elements to move the first and the second structures closer to each other until the quick coupling/uncoupling device closes and to perform coupling of the second end portion of each tubular element with a respective tubular connection sleeve and entering of each elongated element inside a respective coating element.

When the tubular coating element covers a respective elongated element, the magnet is spaced a few millimetres with respect to the bottom wall of the coating element. Said spacing is necessary in order to allow the uncoupling operations that require the first and the second structures to be moved closer together to obtain opening of the quick coupling/uncoupling device without this operation entailing the application of a load by the magnet on the bottom wall of the coating element. Said load could result in uncoupling of the coating element from the tubular sleeve. The presence of the spacing described above prevents attraction of the micro-beads in the terminal part of the coating element on which the micro-beads are not deposited. Said fact reduces the efficiency of the extraction process.

### SUBJECT OF THE INVENTION

The object of the present invention is to provide a device for the loading and the handling of a plurality of coating elements usable in a molecular extraction process, in particular DNA, which solves the drawbacks of the device produced according to the European patent EP2848943.

The preceding object is achieved by the present invention since it relates to a robotized device for the loading and the handling of a plurality of coating elements usable in a molecular extraction process, in particular DNA, of the type described in claim 1.

### BRIEF DESCRIPTION OF THE DRAWINGS

The invention will be illustrated with particular reference to the attached figures which represent a preferred non-limiting embodiment in which:
- figure 1 illustrates, in a perspective view, a robotized device for the extraction of DNA produced according to the teachings of the present invention;
- figures 2 and 3 illustrate, in a perspective view and on an enlarged scale, a detail of the device of figure 1;
- figure 4 illustrates in a frontal view and partially in section the detail illustrated in figures 2 and 3;
- figures 5-15 illustrate operating steps of the device of figure 1;
- figures 16, 17 and 18 illustrate a detail of the device produced according to the present invention.

### PREFERRED EMBODIMENT OF THE INVENTION

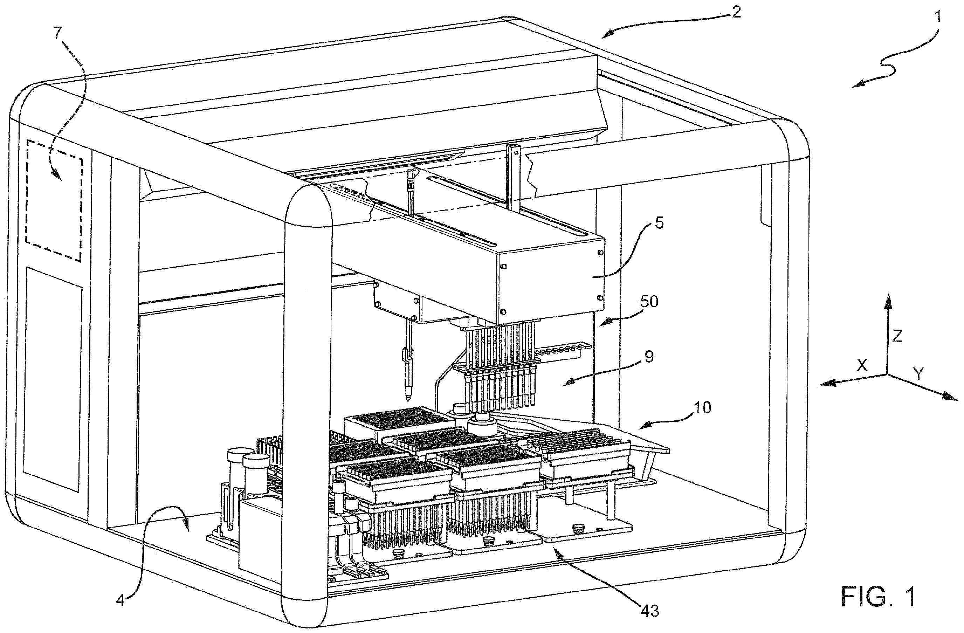

In figure 1 the number 1 indicates, overall, a robotized device for the loading and the handling of a plurality of coating elements usable in a molecular extraction process, in particular DNA.

The device 1 comprises a supporting structure 2 defining a rectangular resting plane 4 accessible frontally and laterally from the outside of the supporting structure 2.

The device 1 further comprises a mobile head 5 which moves along three Cartesian axes X, Y and Z in a parallelepipedal three-dimensional space inside the supporting structure 2 with respect to the rectangular resting plane 4. The head 5 moves under the force of actuators (not illustrated for the sake of simplicity) controlled by an electronic unit 7 to carry out a plurality of operations that will be illustrated below.

The device 1 comprises a picking and conveying structure 9 carried by the head 5 adapted to pick/use/unload a plurality of single-use coating elements 10 made of plastic material. The covering elements, of commercial type, do not form the subject of the present invention.

In particular, during a work cycle, the single-use coating elements 10 are picked and coupled to the picking and conveying structure 9 to coat parts of it so that the robotized device can carry out the work cycle - the coating elements 10 are then conveyed and released at the end of the work cycle by the picking and conveying structure 9 in order to be disposed of.

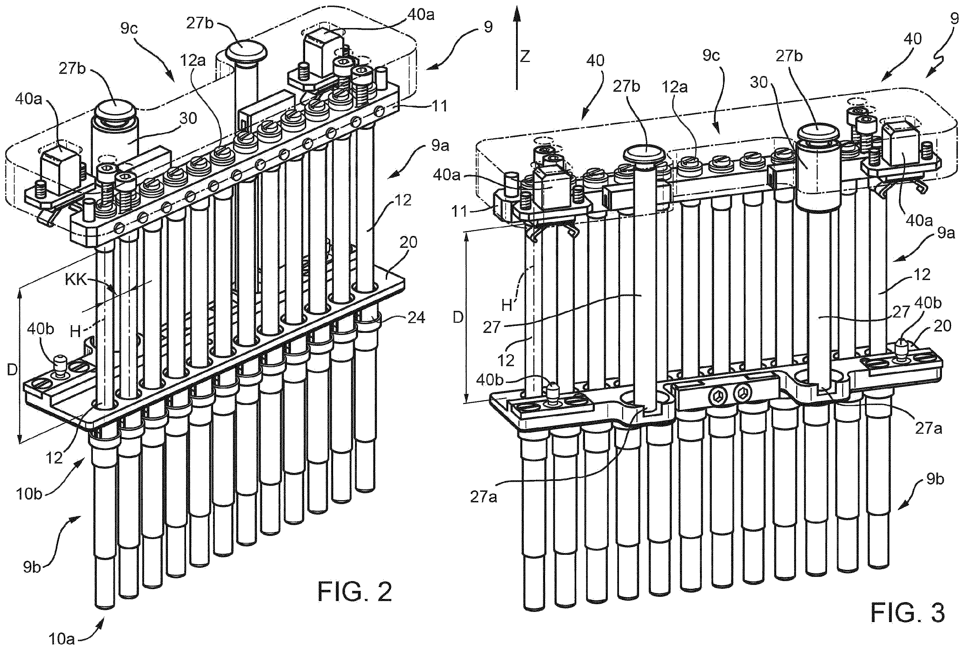

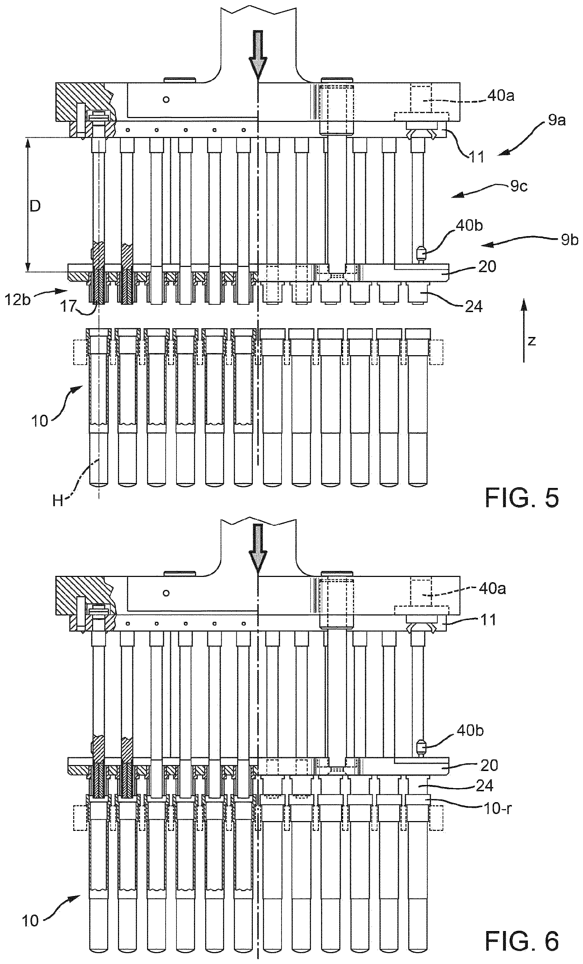

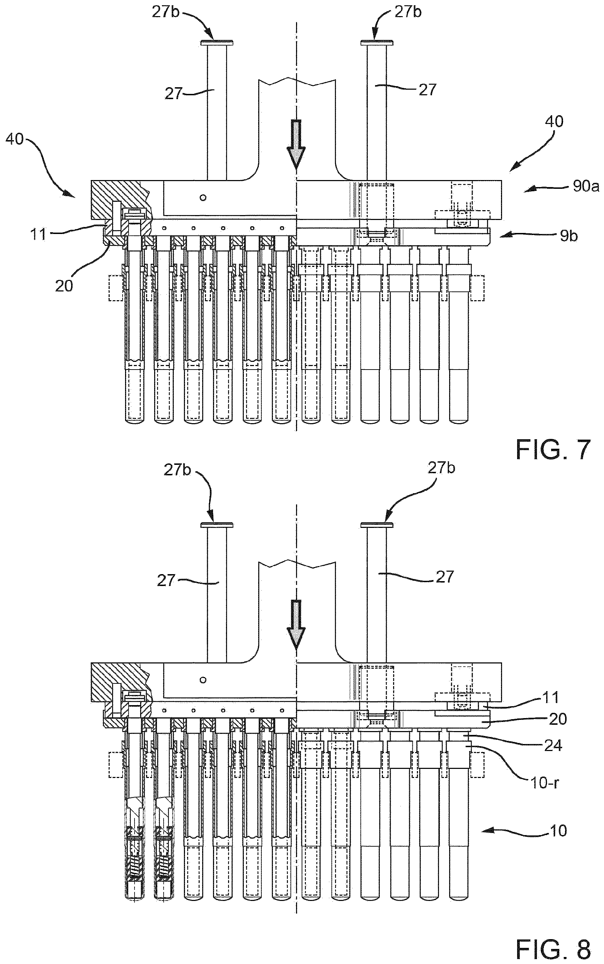

The picking and conveying structure 9 comprises a first upper structure 9a (figure 5) directly carried by the head 5 and a second lower structure 9b mobile with respect to the first upper structure 9a along a guide 9c interposed between the structures 9a and 9b. The guide 9c allows sliding of the lower structure 9b with respect to the upper structure 9a in a vertical direction z between a first stroke end position (figures 7 and 8) in which the first structure 9a is moved close to the second structure 9b and a second stroke end position in which the lower structure 9b is spaced with respect to the upper structure 9a by a predetermined distance D (figures 2 and 3).

In further detail, the first structure 9a comprises a first elongated rectangular flat wall 11 (figures 2 and 3) which carries a plurality of cylindrical elongated elements 12 (rods) arranged with axes H perpendicular to the plane on which the flat wall 11 lies; the axes H of the elongated elements 12 lie on a same vertical plane so that the cylindrical elongated elements 12 form an array of elongated elements coplanar and equispaced from one another along the wall 11 with centre-to-centre distance KK.

The cylindrical elongated elements 12 have the same length L and are made of non-magnetic metallic material, typically aluminium.

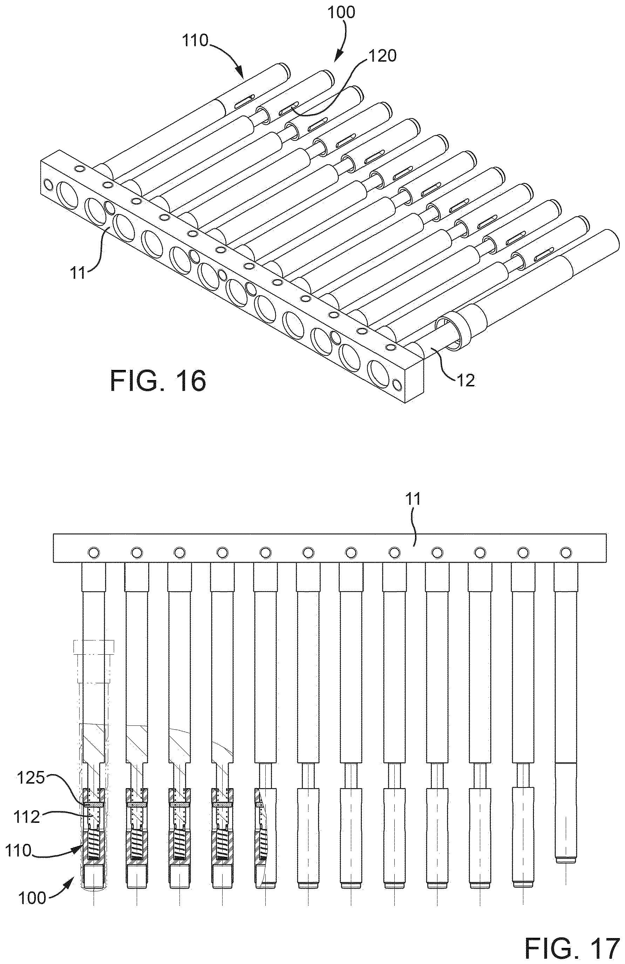

Each cylindrical elongated element 12 has a first upper end portion 12-a firmly fixed (for example by means of screws 16 passing through the wall 11) to the wall 11 and a second lower end portion 12-b which carries a cylindrical permanent magnet 17 (see figures 16, 17 and 18) by means of a support element 100 made according to the present invention and described below.

Also the rectangular wall 11 is made of non-magnetic material, for example aluminium.

In further detail, the permanent magnet 17 has a force direction transverse to the axis H of the elongated element 12 so that the opposite poles of the magnet (N,S) are arranged on the two side portions, namely the diametrically opposite faces of the elongated element 12.

The second lower structure 9b comprises a flat elongated rectangular wall 20 (the wall 20 has substantially the same length as the wall 11 and width slightly greater than the latter, see figure 3) provided with a plurality of through tubular sleeves 24 which extend from the same side of the rectangular wall 20 along an edge of a longer side of the rectangular wall 20.

Each tubular sleeve 24 has an axis coaxial with the axis H of a respective cylindrical elongated element 12 so that - due to the reciprocal arrangement between the lower and upper structures - each cylindrical elongated element 12 is coupled with a through tubular sleeve 24 through which it can slide. The diameter of the through tubular sleeve 24 is in fact larger than the diameter of the cylindrical elongated element 12.

The guide 9c is made of a pair of shafts 27 parallel to each other and each having a first lower end portion 27a (see figure 3) firmly fixed to the rectangular wall 20 and a second widened upper end portion 27b adapted to move into abutment on a bushing 30 inside which the shaft 27 can slide. The bushings 30 are carried by the wall 11 and face upwards; in this way the movement of the wall 20 with respect to the wall 11 is possible due to the sliding of the shafts 27 in the respective bushings 30.

In further detail, at the second stroke end position (figure 3), the end portions 27b move into abutment against the bushings 30 ensuring spacing of the upper structure 9a with respect to the lower structure 9b according to said predetermined distance D.

At the first stroke end position, the walls 20 and 11 are facing and the shafts 27 project from the bushings 30 upwards.

Each single-use coating element 10 comprises a cylindrical tube 10-t (made of plastic material) closed at a first end 10-a by a bottom wall and defining at a second end 10-b a circular opening surrounded by a cylindrical annular edge 10-r. Each single-use coating element 10 has a diameter such as to house an elongated element 12 arranged with its axis H coaxial to the axis of the single-use coating element 10 so that the elongated element 12 is spaced with respect to the inner walls of the coating element 10.

The coating element 10 furthermore has a length substantially equal to L. In this way each single-use coating element 10 is shaped in order to contain a respective elongated element 12 arranged with its axis H coaxial to the axis of the cylindrical tube 10-t.

As will be detailed below, each tubular sleeve 24 is shaped to couple by interference with a free end portion of a respective single-use coating element 10.

In this way the coating elements 10 are arranged, in use when coupled to the second portion 9b, with their axes reciprocally parallel and coplanar to a vertical plane so as to form an array of coating elements carried by the second portion 9b.

A quick coupling/uncoupling device 40 (of known type) is also provided interposed between the first upper structure 9a and the second lower structure 9b.

The quick coupling/uncoupling device 40 - according to the embodiment example illustrated - comprises two units arranged at the end of the walls 11/20; each unit comprises a first member 40-a carried by the wall 11 and a second member 40-b carried by the wall.

The first and the second members 40-a, 40-b - of known type - are adapted to snap couple with each other when they are pressed against each other, namely when the first upper structure 9a is pushed towards the second lower structure 9b. Following the snap coupling, the walls 11 and 10 are maintained reciprocally connected, side-by-side. Following a further pressure of the first upper structure 9a towards the second lower structure 9b, the first and second members 40-a, 40-b uncouple from each other.

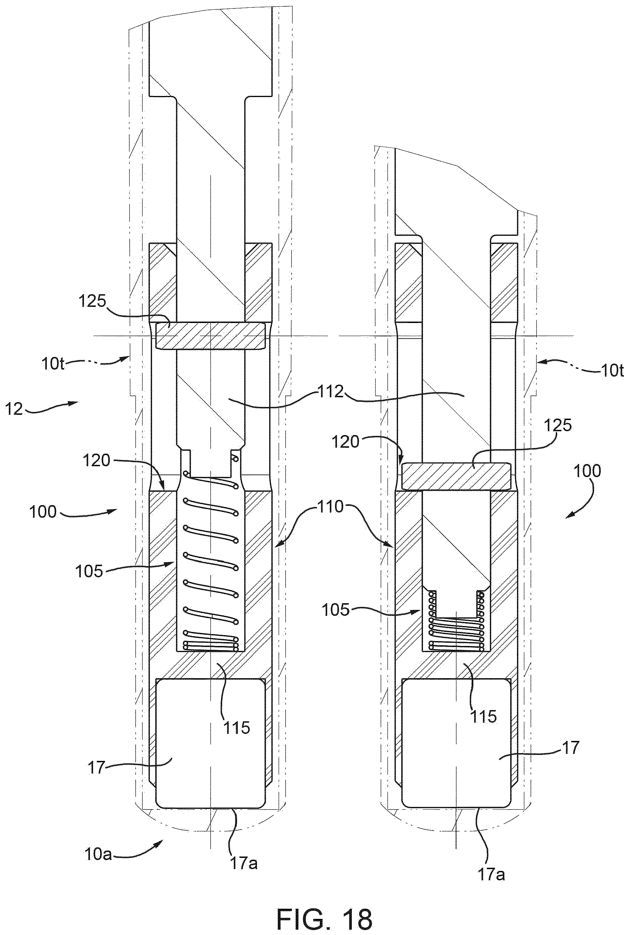

According to the present invention (figures 16, 17 and 18), each cylindrical elongated element 12 is provided with a support element 100 interposed between a free end portion of the elongated element 12 and the cylindrical permanent magnet 17 and adapted to allow a bidirectional axial movement of the magnet 17 along the axis H with respect to the elongated element 12. The support element 100 is provided with an elastic element 105 adapted to maintain the magnet 17 in a rest position in which the flat free end 17-a of the magnet 17 has a maximum distance F with respect to the first upper structure 11. Said elastic element 105 is compressible when the end portion 17-a of the magnet presses on the bottom wall of the tubular element 10 (figure 18 right-hand side) so that the distance between the free end 17-a of the magnet 17 and the first upper structure 11 assumes a value lower than F.

In further detail, the support element 100 comprises a cylindrical tubular portion 110 which is adapted to slide axially along a cylindrical portion having reduced diameter 112 of the elongated element 12 and a helical spring which provides the elastic element 105. The spring 105 extends axially and is interposed between the end of the cylindrical portion having reduced diameter 112 and a partition 115 which extends transversally to the cylindrical tubular portion 110. The cylindrical tubular portion 110 is provided with a pair of slots 120 which extend in an axial direction and which house end portions of a pin 125 which extends transversally to the cylindrical portion having reduced diameter 110. The pin 125 moves into abutment against an end of the slots 120 defining the maximum expansion of the spring 105 and preventing detachment of the cylindrical tubular portion 110 from the cylindrical elongated element 12.

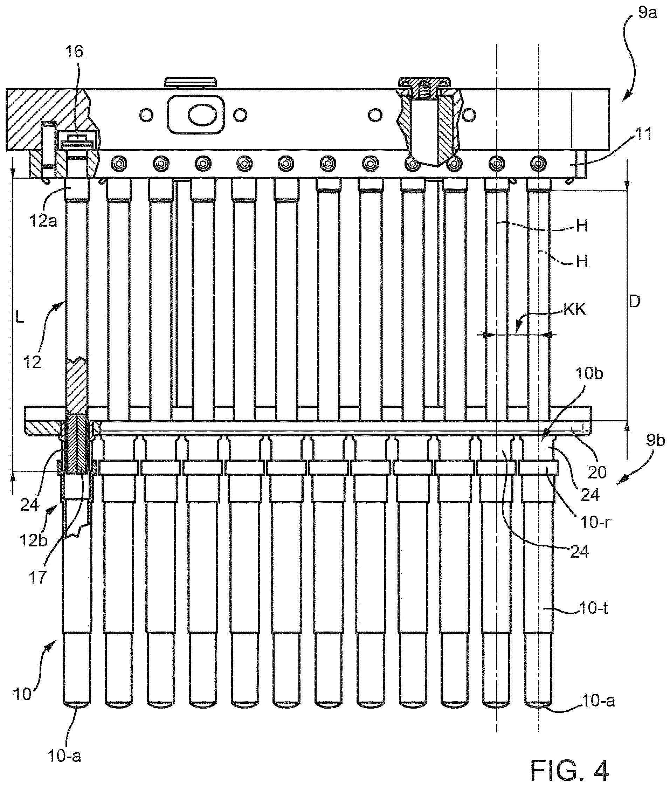

In use, at the beginning of a work cycle (figure 3), the lower structure 9b is arranged in the second stroke end position in which the lower structure 9b is spaced with respect to the upper structure 9a by the predetermined distance D (see figure 4). The walls 11 and 20 are therefore spaced by the distance D.

The head 5 moves along x and y under the control of the electronic unit 7 until it is arranged above a plurality of support elements arranged on the resting plane 4 (typically carried by housing carrier elements 43 of known type which arrange the single-use coating elements 10 according to a matrix structure).

The positioning of the control unit 7 allows each element 12 to be arranged with its axis coaxial to the axis of a respective single-use coating element 10; at said point the motion of the head along x, y terminates and it is lowered along z (figure 6).

The lowering of the head 5 causes in sequence:
a) entering of the tubular sleeves 24 inside the respective single-use coating elements 10 (figure 6) for a first stretch f1 - the coupling between the tubular elements 24 prevents the wall 10 from moving farther down, whereas the wall 11 continues to move down (the shafts 27 slide in the bushings 30) and closer to the wall 20 resting on the coating elements 10;
b) arrangement of the wall 11 in contact with the wall 20 and consequent closing of the quick coupling/uncoupling device 40 due to the pressure exerted by the wall 11 on the wall 20 (figure 7) ;
c) following permanent coupling between the two walls 11 and 20 the tubular sleeves 24 penetrate farther (see figure 8) into the respective coating elements 10 for a second stretch f2 greater than the stretch f1 ensuring a stable coupling between the second lower portion 9b and the single-use coating elements 10.

Each cylindrical elongated element 12 penetrates into a respective coating element 10 and the flat end portion 17-a of the magnet 17 is arranged in contact with the bottom wall of the coating element 10 - in said position the spring 105 is not subject to pressure. The distance between the free end 17-a of the magnet 17 and the first upper structure 11 assumes a value equal to F.

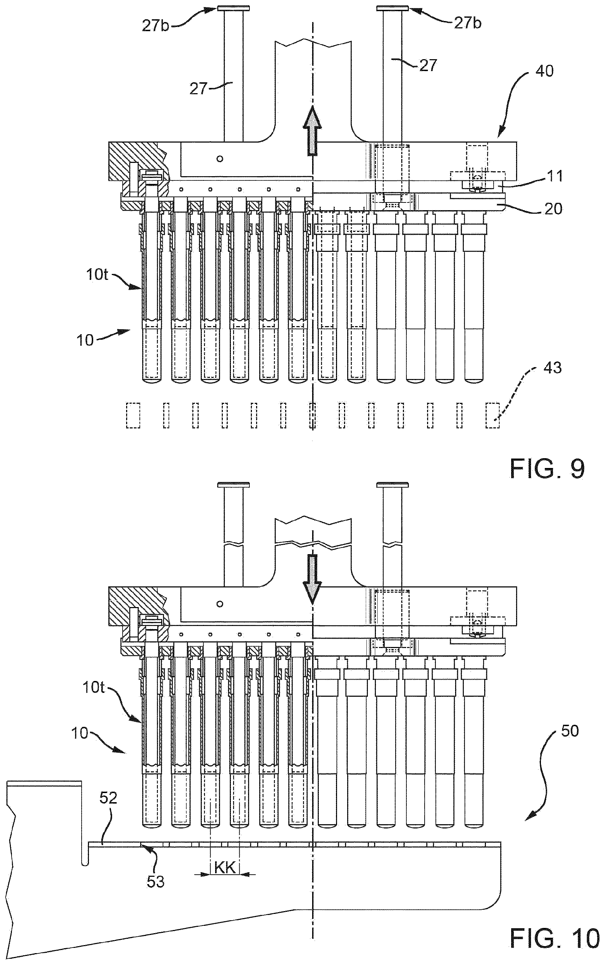

The head 5 is subsequently raised (figure 9 movement along z) to pick the coating elements 10 from the housing carrier elements 43 and subsequently moved in order to carry out the DNA extraction operations according to techniques known in the literature and therefore briefly mentioned.

Said operations can comprise:
a) lysis of the cells by means of reagent - the lysis operation is carried out in a plurality of wells (12 samples: 1 sample/patient) ;
b)insertion into each well of the magnetic micro-beads coated in material adapted to absorb the DNA;
c) picking of the coating elements 10 based on the above operations;
d) insertion into the well of the permanent magnet 17 coated in the coating element 10 so that the magnetic micro-beads deposit on the outer surface of the coating element 10;
e) extraction of the coating element 10 from the well and insertion in a well containing a washing fluid - the coating element is moved in alternating directions inside the well containing the washing fluid; and
g) elution of the DNA.

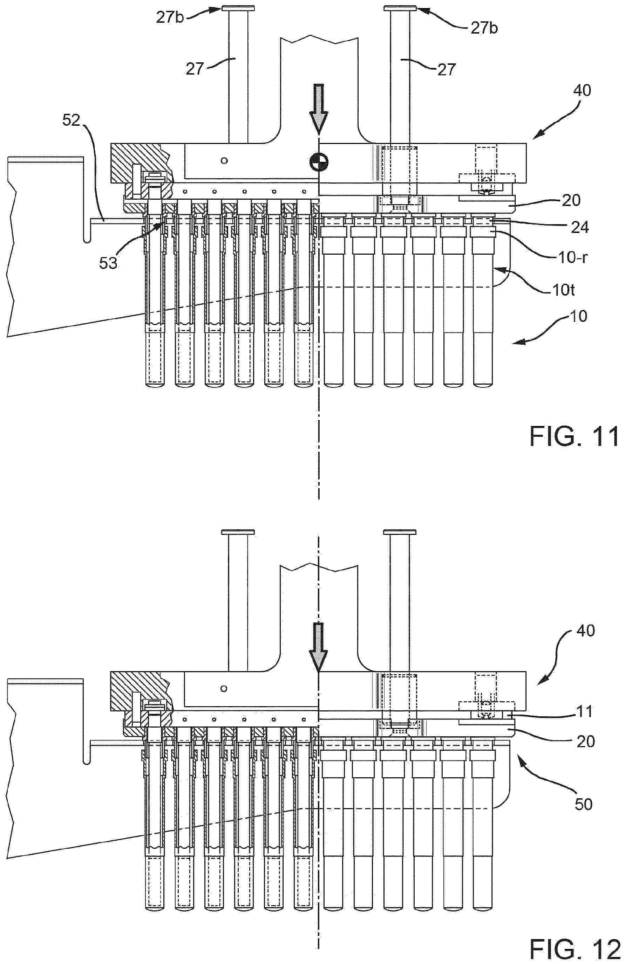

To carry out the decoupling operations of the coating elements 10 from the picking and conveying structure 9 a tool 50 is used (figures 11 and 12) carried by a vertical wall of the supporting structure 2.

The tool 50 comprises an elongated flat rectangular wall 52 provided with a plurality of openings 53 which open along a longer side of the flat elongated wall 52. The openings 53 have an approximately circular shape with axes reciprocally spaced by a distance equal to the centre-to- centre distance KK; the diameter of said openings 53 is equal to the external diameter of the tube 10-t but is smaller than the external diameter of the cylindrical annular edge of the coating element 10.

To perform the decoupling operations, the head 5 moves under the control of the electronic unit 7 arranging (figure 11) the portion of the tubular sleeves 24 coated by the tube 10-t inside a respective opening 53; in this way, an upper face of the wall 52 faces the wall 20 while the cylindrical annular edges 10-r of the coating elements 10 face a lower face of the wall 52.

Subsequently the head 5 is further lowered (figure 12) for a stretch causing a pressure of the wall 11 on the wall 20 which rests on the tool 50; said operation contributes to opening of the quick coupling/uncoupling device 40. During said compression operation, the flat end portion 17-a of the magnet 17 exerts a pressure against the bottom wall of the coating element 10 - the spring 105 is compressed maintaining the magnet 17 in contact with the bottom wall of the coating element 10, the cylindrical tubular portion 110 slides along the cylindrical portion having reduced diameter 112 and the distance between the free end 17-a of the magnet 17 and the first upper structure 11 assumes a value lower than F.

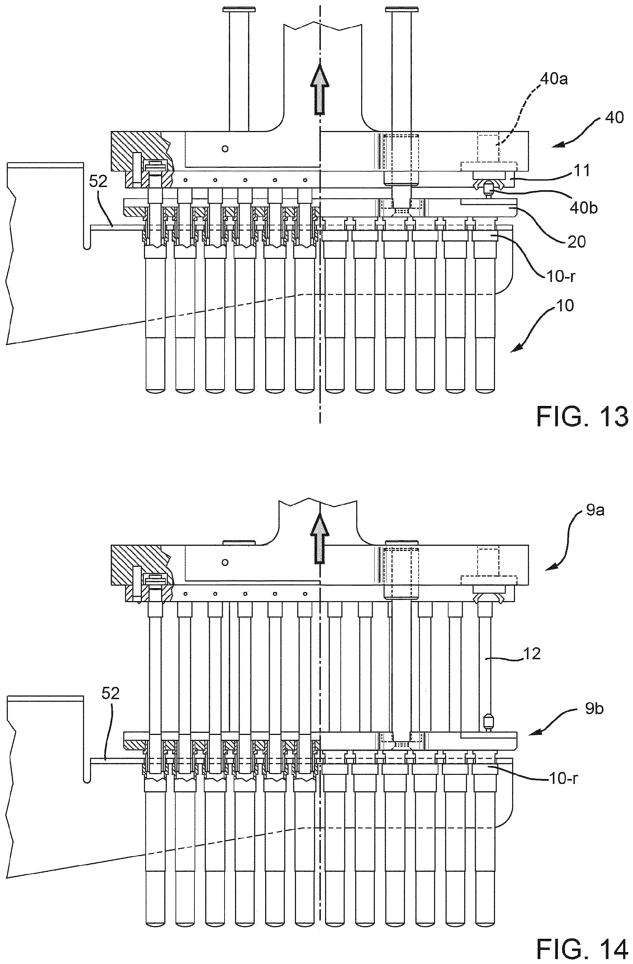

The head is then raised so that the wall 11 (figure 13) is distanced from the wall 20 which is weakly coupled to the tool 50 due to the interference exerted by the cylindrical annular edges of the coating elements 10 on the lower face of the wall 52.

When the wall 11 reaches with respect to the wall 20 the stroke end position in which the lower portion 9b is spaced with respect to the upper portion 9a by a predetermined distance D, the cylindrical annular edges 10-r of the coating elements 10 move into abutment against the lower face of the wall 52 (figure 14).

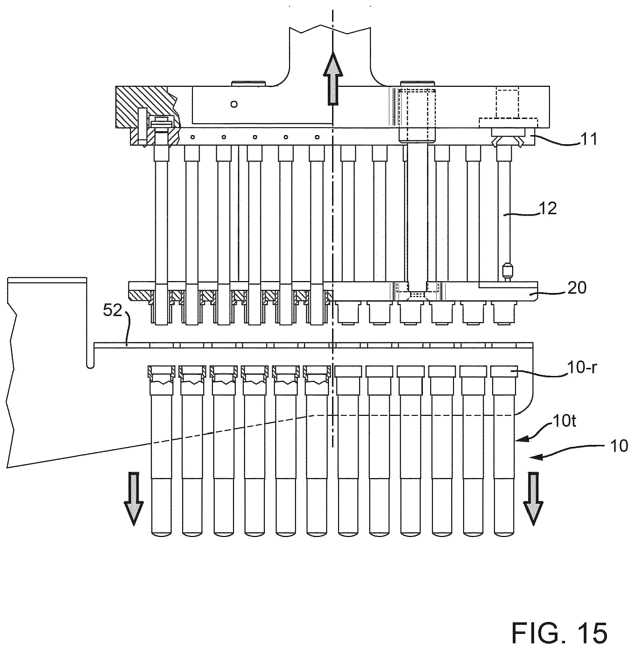

A subsequent upstroke of the head (figure 15) causes the tubes 10-t to come out of the tubular sleeves 24 allowing release by gravity of the single-use coating elements 10.

The picking and conveying structure 9 then returns to the arrangement it had at the beginning of the work cycle. The operations previously illustrated can then be repeated for loading of a subsequent batch of coating elements 10.

As can be seen from the preceding description, during all the operations illustrated above, the permanent magnet 17 is always arranged in contact with the bottom wall of the coating element 10 and the cylindrical element extends throughout the length of the coating element.

Said arrangement of the magnet allows a greater number of magnetic particles to be attracted than the number of magnetic particles attracted by a device produced according to the patent EP-B-2.848.943 of the applicant which requires a spacing of a few millimetres between the magnet and the bottom wall of the coating element.

## Claims

1. A robotized device for the loading and the handling of a plurality of single-use coating elements (10) usable in a molecular extraction process, in particular DNA, comprising:
- a mobile head (5) arranged in a three-dimensional space with respect to a plane on which the device rests, under the force of actuators controlled by an electronic unit (7);
- a picking and conveying structure (9) carried by the head (5) adapted to pick/use/unload the single-use coating elements (10) each of which comprises a tubular element (10-t) closed at a first end (10-a) by a bottom wall and defining at its second end (10-b) a circular opening surrounded by a circular annular edge (10-r),
the picking and conveying structure (9) comprises a first upper structure (9a) directly carried by the head (5) and a second lower structure (9b) mobile with respect to the first upper structure (9a) along a vertical guide (9c) interposed between the first and the second structures (9a, 9b) between a first stroke end position in which the first structure (9a) is arranged side-by-side with the second structure (9b) and a second stroke end position in which the lower structure (9b) is spaced, with respect to the upper structure (9a), by a predetermined distance D;
- a plurality of elongated elements (12) carried by the upper structure (9a) arranged with first axes parallel and coplanar to one another so as to form an array of elongated elements spaced from one another by a centre-to-centre distance KK; each elongated element being configured to carry at one of its ends a respective magnetic element (17);
- a plurality of connection sleeves (24) carried by the lower structure (9b) and each adapted to be coupled to a respective tubular element (10-t);
- quick coupling/uncoupling means (40) interposed between the first upper structure (9a) and the second lower structure (9b); said robot being adapted to carry out a first positioning step of said head (5) with respect to said coating elements for achieving the approaching between the first and the second structures until closing the quick coupling/uncoupling means (40) and performing the coupling of the second end portion of each tubular element (10-t) with a respective connection sleeve (24) and the entering of each elongated element (12) inside a respective coating element,
**characterised in that** each elongated element (12) is provided with a support element (100) interposed between a free end portion of the elongated element (12) and the magnet (17) and adapted to allow a bidirectional axial movement of the magnet (17) with respect to the elongated element (12); the support element (100) is provided with an elastic element (105) adapted to keep the magnet (17) in a rest position in which the free end (17-a) of the magnet (17) is arranged facing, in particular in contact with, said bottom wall and having a distance F with respect to the first upper structure (11); said elastic element (105) is compressible when the end portion (17-a) of the magnet presses against the bottom wall of the tubular element (10) and the distance between the free end (17-a) of the magnet (17) and the first upper structure (11) assumes a value lower than F.

2. The device according to claim 1, wherein the support element (100) comprises a cylindrical tubular portion (110) that is configured to axially slide along a cylindrical portion having a reduced diameter (112) of the elongated element (12) and a helical spring that constitutes the elastic element (105); the spring (105) axially extends and is interposed between an end of the cylindrical portion having a reduced diameter (112) and a partition (115) that extends transversally to the cylindrical tubular portion (110).

3. The device according to claim 2, wherein the cylindrical tubular portion (110) is provided with at least a slot (120) that extends in an axial direction and houses an end portion of a pin (125) that extends transversally to the cylindrical portion having a reduced diameter (110); the pin (125) is configured to move into abutment against an end of the slot (120) defining the maximum expansion of the spring (105) and preventing the detachment of the cylindrical tubular portion (110) from the cylindrical elongated element (12).

4. The device according to one of the preceding claims, wherein said robot is adapted to carry out, after said quick coupling/uncoupling means are closed, a further travel of the first and the second structure, coupled to each other towards the coating elements (10) in order to obtain a further and more stable coupling of the coupling walls (24) with the respective coating elements (10).

5. The device according to claim 4, wherein each sleeve extends through a wall (20) of the lower structure; said sleeve (24) being adapted to be coupled, by interference, with the end portion of a respective tubular element (10-t);
said first and second structures being mutually configured and arranged so that each elongated element engages a tubular wall for penetrating the inside of a respective coating element during said first step.

6. The device according to one of the preceding claims, wherein said guide comprises a pair of rectilinear elements having first ends firmly connected to the second lower structure (9b), engaging bushings carried by the second structure and having second end portions adapted to move into abutment against said bushings at said second stroke end position.

7. The device according to any one of the preceding claims, wherein all of said elongated elements (12) have the same length (L).

8. The device according to any one of the preceding claims, wherein said elongated elements (12) have a cylindrical shape.

9. The device according to any one of the preceding claims, wherein said elongated elements (12) are made of a non-magnetic metal material, typically aluminium.

10. The device according to any one of the preceding claims, wherein the permanent magnet has a force direction transversal to the axis of the elongated element (12) so that said opposite poles of the magnet (N, S) are provided on diametrically opposite faces of the elongated element (12).

11. The device according to any one of the preceding claims, wherein the device is provided with an unloading tool (50) carried by a wall of the support structure (2) and comprising an elongated wall (52) provided with a plurality of release openings (53) which open along a longer side of the elongated wall (52); said release openings have axes spaced from one another by a distance equal to the centre-to-centre distance KK; the diameter of said release openings (53) being smaller than the outer diameter of the cylindrical annular edge of the coating element (10);
said electronic unit (7) being configured to move said head and arrange (figure 12) the portion of the connection walls not coupled to a respective tubular element inside a respective release opening (53) so that an upper face of the elongated wall (52) is facing the lower structure (9b) while the annular edges (10-r) of the coating elements (10) are facing a lower face of the elongated wall (52);
said electronic unit (7) being configured to:
- move the head (5) towards said unloading tool (50) for achieving the opening of the quick coupling/uncoupling means (40) and subsequently;
- distance the head (5) from the unloading tool so that the first and the second structures are spaced further from the unloading tool and the annular edges (10-r) of the coating elements (10) move into abutment against said lower face and the coating elements (19) uncouple from the connection walls and are released, due to gravity, from the picking and conveying structure.

12. A system according to any one of the preceding claims, wherein said quick coupling/uncoupling means comprise a first member carried by the first support element and a second member carried by the second support element and adapted to be snap coupled with the first member when the first support element is moved closer and pressed onto the second support element; after the snap coupling has been achieved, the pressure applied on the first support element causes the uncoupling of the first and the second member.
